# FASCICULE DE BREVET EUROPEEN

(11) **EP 0 511 905 B1**
(45) Date de publication et mention de la délivrance du brevet: **23.08.1995**
(21) Numéro de dépôt: 92401194.3
(22) Date de dépôt: 24.04.1992
(51) Int. Cl.: A61F 13/15

(54) **Serviette périodique à souflets latéraux**
Monatsbinde mit Seitenteilen
Sanitary napkin with lateral flaps

(30) Priorité: 29.04.1991 FR 9105252
(43) Date de publication de la demande: 04.11.1992
(73) Titulaire: KAYSERSBERG SA, F-68240 Kaysersberg (FR)
(72) Inventeur: Pigneul, Raymond, F-68320 Durrenentzen (FR)
(74) Mandataire: David, Daniel

(56) Documents cités:
- EP-A- 0 374 640
- EP-A- 0 405 403
- FR-A- 2 604 608
- US-A- 3 693 621

## Description

La présente invention concerne une protection périodique et plus particulièrement une serviette hygiénique à volets ou soufflets latéraux ayant une protection améliorée contre les fuites latérales.

On connaît déjà d'après l'EP 134086 une serviette périodique ou similaire, en particulier pour l'hygiène féminine, comprenant un élément tampon 216 absorbant les fluides disposé entièrement à l'intérieur d'une enveloppe 214-230-234 dont au moins la partie 214 de réception des fluides est perméable aux fluides. Des volets latéraux 224, 224′ sont prévus pour améliorer l'étanchéité latérale par le fait que ces volets latéraux sont disposés à l'intérieur du sous-vêtement et ont leurs extrémités repliées autour des bords latéraux 246 ou 246′ de ce sous-vêtement.

L'EP-A 130848 décrit un enseignement similaire, selon lequel en outre les volets latéraux peuvent également contenir à l'intérieur un élément tampon absorbant les fluides.

Il a pu être observé que ces volets latéraux étaient d'une dimension insuffisante pour protéger correctement l'ensemble du linge de corps (voir également EP-A-405403).

En outre, les serviettes périodiques une fois usagées sont en général jetées en étant introduites dans un sachet jetable livré séparément avec les serviettes, ce qui constitue une complication d'emploi, et augmente les coûts de fabrication (voir FR-A-2604608).

La présente invention a pour objet de fournir une serviette périodique présentant une protection suffisante contre les fuites latérales permettant de protéger correctement le linge de corps.

L'invention a également pour objet de résoudre le problème technique consistant en la fourniture d'une solution permettant d'éviter l'emploi d'un emballage distinct pour jeter la serviette périodique après usage.

D'autres objets et avantages de la présente invention apparaîtront à la lecture de la description ci-après.

Selon l'invention, il est fourni une serviette périodique destinée à être portée avec un sous-vêtement de maintien ayant une partie entrejambe, comportant :
a) un moyen absorbant de forme allongée ayant deux bords opposés longitudinaux, deux bords opposés transversaux, une face supérieure perméable aux fluides corporels, au contact du corps et une face inférieure au contact du sous-vêtement ;
b) deux volets ou soufflets latéraux s'étendant le long desdits bords longitudinaux sur une longueur sensiblement égale à celle dudit moyen absorbant,
caractérisée en ce que lesdits volets ou soufflets latéraux sont liés audit moyen absorbant par une liaison sur ladite face supérieure et en ce qu'ils sont munis à chacune de leurs extrémités libres d'un élément d'adhérence permettant leur fixation au sous-vêtement.

Selon un premier mode de réalisation de l'invention la liaison de chacun des volets ou soufflets latéraux sur la face supérieure du moyen absorbant est effectué dans la partie centrale dudit moyen absorbant à proximité de ses bords longitudinaux respectifs. Dans ce cas ladite liaison est avantageusement effectuée sur une longueur d'environ 3 cm à environ 8 cm.

Selon un deuxième mode de réalisation de l'invention la liaison des volets ou soufflets latéraux à la face supérieure du moyen absorbant est effectuée à l'une des extrémités du moyen absorbant à proximité de l'un des bords transversaux au moyen absorbant.

La liaison des volets ou soufflets latéraux à la face supérieure du moyen absorbant peut être effectuée par tout moyen,, par exemple par collage.

La présente invention sera maintenant décrite en référence aux Figures annexées dans lesquelles :
La Figure 1 est une vue schématique de dessus d'une serviette périodique selon le premier mode de réalisation de l'invention dans laquelle les volets ou soufflets latéraux sont dépliés.
La Figure 2 est une vue schématique de dessus d'une serviette périodique selon le premier mode de réalisation de l'invention dans laquelle les volets ou soufflets latéraux sont repliés.
La Figure 3 est une vue schématique de dessus d'une serviette périodique selon une variante du premier mode de réalisation de l'invention.
La Figure 4 est une vue schématique de dessus d'une serviette périodique selon une variante du premier mode de réalisation de l'invention.
La Figure 5 est une vue schématique de dessus d'une serviette périodique selon le deuxième mode de réalisation de l'invention dans laquelle les volets ou soufflets latéraux sont dépliés.
La Figure 6 est une vue schématique de dessus d'une serviette périodique selon une variante du deuxième mode de réalisation de l'invention dans laquelle les volets ou soufflets latéraux sont dépliés.
La Figure 7 est une vue schématique de dessus de la serviette périodique de la Figure 4 dans laquelle les volets ou soufflets sont repliés.
La Figure 8 est une vue schématique partielle d'un slip classique dans lequel est positionnée une serviette périodique selon le deuxième mode de réalisation de l'invention.
La Figure 9 est une vue schématique partielle d'un slip étroit ou "string" dans lequel est positionnée une serviette périodique selon le deuxième mode de réalisation de l'invention.
La Figure 10 est une vue en coupe transversale selon la ligne de tracé I-I′ de la Figure 3.

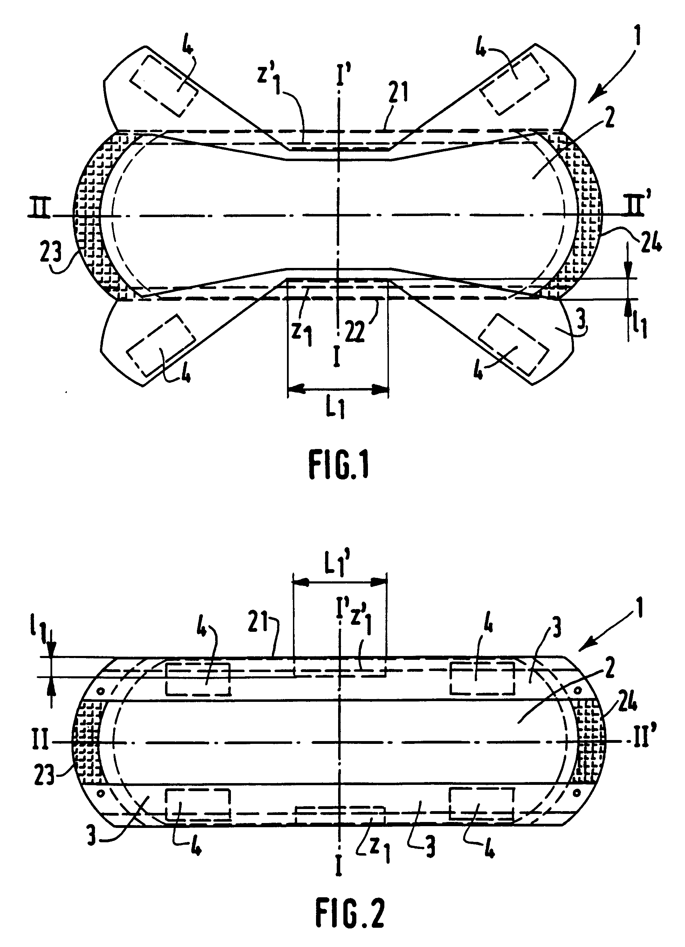

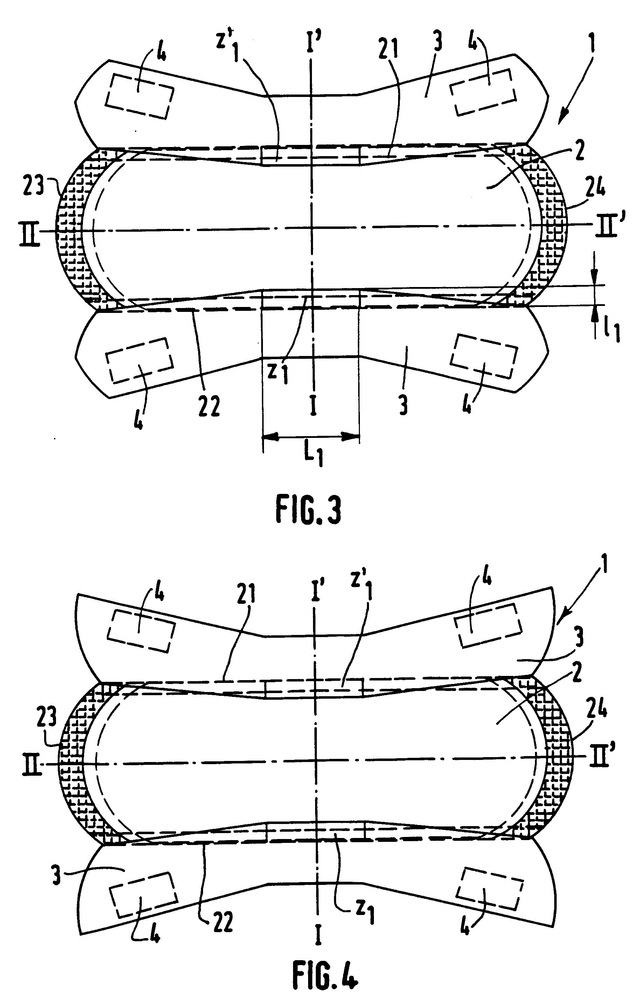

En référence aux Figures 1-4, on a représenté une serviette périodique à volets ou soufflets latéraux. Cette serviette est représentée par le numéro de référence général 1 et comprend un moyen absorbant de forme allongée 2 délimité par deux bords longitudinaux opposés 21 et 22 et deux bords transversaux opposés 23 et 24. Les volets ou soufflets latéraux 3 sont liés sur la face supérieure du matelas absorbant dans les zones respectives z₁ et z′₁ situées dans la partie centrale du moyen absorbant 2 à proximité des bords longitudinaux respectifs 21 et 22. Les zones z₁ et z₁′ sont délimitées respectivement par les dimensions longitudinales L₁ et L′₁ et par les dimensions transversales 1₁ et 1₁′. Chacune des dimensions longitudinales L₁ et L₁′ est comprise de préférence dans l'intervalle d'environ 3 cm à environ 8 cm.

Chacune des extrémités libres des volets ou soufflets latéraux 3 est munie d'un élément d'adhérence 4. Les éléments d'adhérence 4 sont provisoirement protégés par un moyen de protection amovible (non représenté) tel une bande de protection détachable, par exemple une bande de papier siliconé. Les éléments d'adhérence 4 sont fixés sur la face des volets ou soufflets latéraux 3 qui est la face supérieure quand ces derniers sont en position repliée, comme indiqué à la Figure 2.

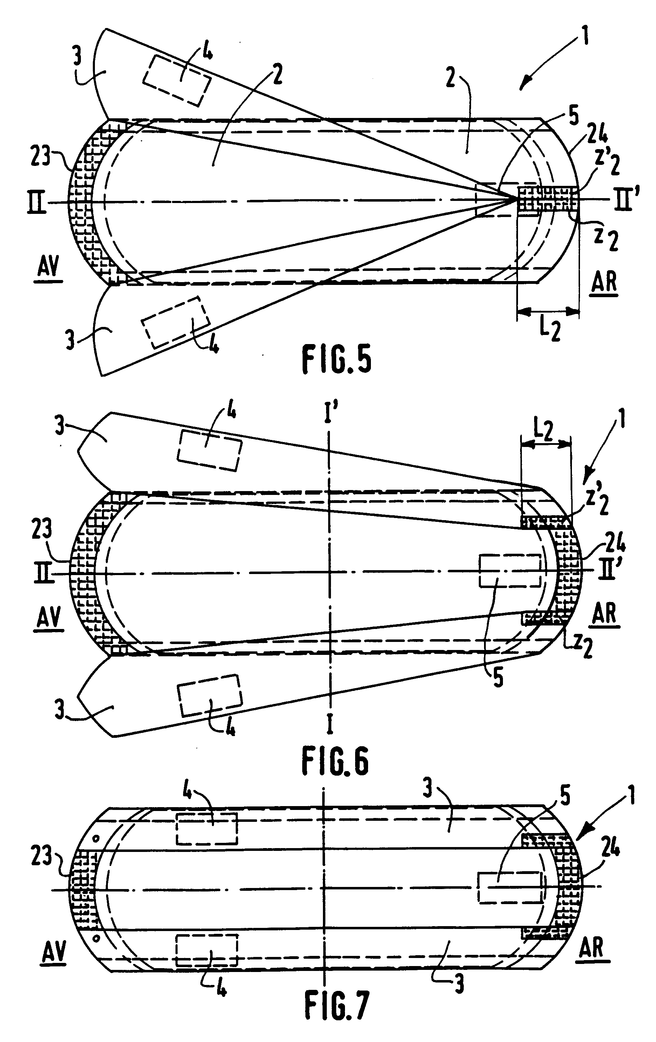

Les Figure 5-7 illustrent un deuxième mode de réalisation de l'invention dans lequel chacun des volets ou soufflets latéraux 3 est lié sur la face supérieure du matelas absorbant 2 à l'une des extrémités de ce dernier, qui est l'extrémité arrière AR lors de l'utilisation de la protection périodique, dans les zones respectives z₂ et z₂′. Les zones z₂ et z₂′ peuvent être juxtaposées dans la partie médiane de l'extrémité AR, comme illustré par la Figure 5, ou bien symétriques par rapport à l'axe longitudinal II-II' du matelas absorbant 2 comme illustré par les Figures 6 et 7. A titre d'exemple, les zones Z₂ et Z₂′ peuvent avoir une dimension longitudinale L₂ d'environ 2 cm.

Chacune des extrémités libres des volets ou soufflets latéraux 3, qui se trouvent à la partie avant AV du matelas absorbant 2 est munie d'éléments d'adhérence 4. Les éléments d'adhérence 4 sont fixés sur la face des volets ou soufflets latéraux 3 qui est la face supérieure quand ces derniers sont en position repliée. Le moyen absorbant 2 est muni sur sa face intérieure, côté sous-vêtement, d'un élément d'adhérence 5.

Les éléments d'adhérence 4 et 5 sont provisoirement protégés par un moyen de protection amovible (non représenté) tel une bande de protection détachable, par exemple une bande de papier siliconé.

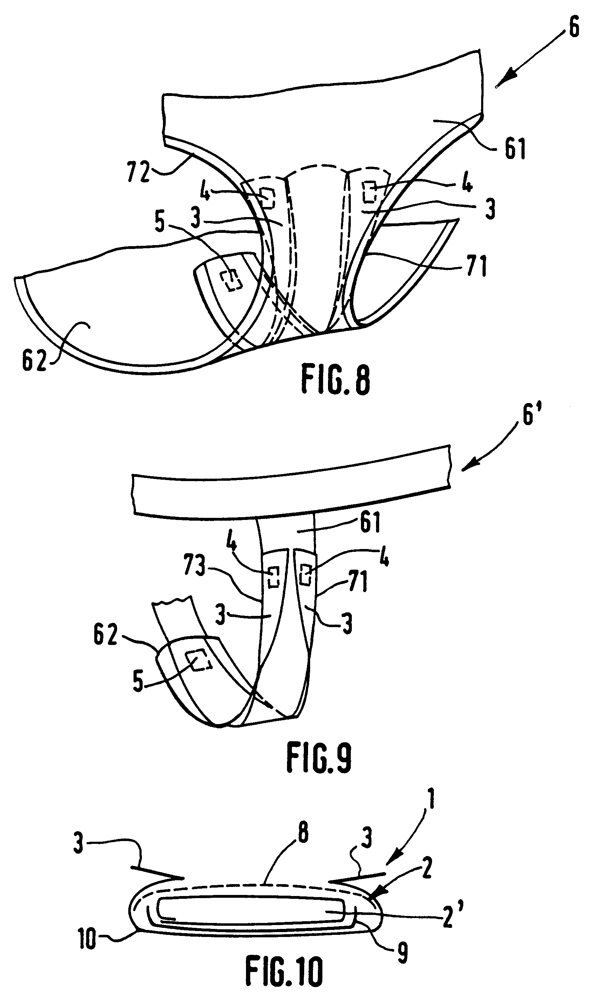

En référence aux Figures 8 et 9, on a représenté un slip, référencé respectivement 6 et 6′, ayant une partie avant 61 et une partie arrière 62, et des bords latéraux 71 et 72 dans la partie entrejambe, dans lequel est positionnée une serviette périodique selon le deuxième mode de réalisation de l'invention illustré par les Figures 6 et 7.

La Figure 8 illustre un slip de taille normale dans lequel les éléments d'adhérence 4 des volets ou soufflets latéraux 3 sont fixés sur la partie avant 61 interne du slip. L'élément d'adhérence 5, fixé sur la partie arrière 62 interne du slip, contribue au positionnement correct de la serviette périodique dans le slip.

La Figure 9 illustre un slip étroit ou "string" 6′ dans lequel les éléments d'adhérence 4 des volets ou soufflets latéraux 3 sont fixés sur la partie avant 61 externe du slip, les volets ou soufflets latéraux 3 ayant été repliés autour des bords latéraux 71 et 72 du slip. L'élément d'adhérence 5, fixé sur la parie arrière 62 interne du slip contribue au positionnement correct de la serviette périodique dans le slip.

Les positionnements dans des slips d'une serviette périodique selon le premier mode de réalisation de l'invention illustré par les Figures 1-4 n'ont pas été représentés. Ils sont similaires à ceux illustrés dans les Figures 8 et 9 sauf que la serviette périodique peut être fixée sur le slip au moyen des quatre éléments d'adhérence 4 des volets ou soufflets latéraux 3 dans un sens arbitraire, la serviette périodique n'ayant pas de partie arrière et avant spécifiques.

En référence à la Figure 10, on a représenté une serviette périodique 1 comprenant un moyen absorbant 2 constitué par un matelas absorbant 2′ comportant sur sa face supérieure, au contact du corps, une voie 8 perméable aux fluides corporels et sur sa face inférieure une feuille de fond 9 imperméable. Le moyen absorbant 2 est partiellement entouré par une enveloppe 10 dans laquelle sont formés les volets ou soufflets latéraux 3.

Les volets ou soufflets latéraux 3 peuvent également être formés indépendamment et reliés par des techniques connues de l'homme de métier.

Quand la serviette périodique est mise en place, que ce soit dans un slip de taille normale, comme illustré par la Figure 8, ou dans un slip étroit ou "string", comme illustré dans la Figure 9, il y a formation d'une "coquille" enveloppant l'orifice vaginal et protégeant de ce fait le sous-vêtement contre les fuites latérales.

Après usage, les volets ou soufflets latéraux 3 et leurs éléments d'adhérence 4 permettent d'envelopper la serviette avant de la jeter.

## Revendications

1. Serviette (1) périodique destinée à être portée avec un sous-vêtement de maintien ayant une partie entrejambe, comportant :
a) un moyen absorbant (2) de forme allongée ayant deux bords opposés longitudinaux (21,22), deux bords opposés transversaux (23,24), une face supérieure perméable aux fluides corporels au contact du corps, et une face inférieure côté sous-vêtement,
b) deux volets (3) ou soufflets latéraux (3) s'étendant le long desdits bords longitudinaux dudit moyen absorbant,
caractérisée en ce que lesdits volets ou soufflets latéraux ont une longueur sensiblement égale à celle dudit moyen absorbant, en ce qu'ils sont liés audit moyen absorbant par une liaison sur ladite face supérieure et en ce qu'ils sont munis à chacune de leurs extrémités libres d'un élément d'adhérence (4) permettant leur fixation au sous-vêtement.

2. Serviette périodique selon la revendication 1, caractérisée par le fait que ladite liaison de chacun desdits volets ou soufflets latéraux sur ladite face supérieure dudit moyen absorbant est effectuée dans la partie centrale dudit moyen absorbant à proximité des bords longitudinaux respectifs dudit moyen absorbant.

3. Serviette périodique selon la revendication 2, caractérisée par le fait que ladite liaison est effectuée sur une longueur d'environ 3 cm à environ 8 cm.

4. Serviette périodique selon la revendication 1, caractérisée par le fait que la liaison desdits volets ou soufflets latéraux sur ladite face supérieure dudit moyen absorbant est effectuée à l'une des extrémités dudit moyen absorbant à proximité de l'un de ses bords transversaux.

## Claims

1. Sanitary towel (1) intended to be worn with a supporting undergarment with a crotch part, including:
a) an elongate absorbent means (2), with two opposite longitudinal edges (21, 22), two opposite transverse edges (23, 24), a top face permeable to body fluids, in contact with the body, and a bottom face on the undergarment side.
b) two lateral wings (3) or flaps (3) extending along the said longitudinal edges of the said absorbent means,
characterised in that the said lateral wings or flaps have a length substantially equal to that of the said absorbent means, in that they are joined to the said absorbent means by a connection on the said top face and in that they are provided with an adherent element (14) at each of their free ends enabling them to be fixed to the undergarment.

2. Sanitary towel according to Claim 1, characterised in that the said connection of each of the said lateral wings or flaps to the said top face of the said absorbent means is effected in the central part of the said absorbent means close to the respective longitudinal edges of the said absorbent means.

3. Sanitary towel according to Claim 2, characterised in that the said connection is effected over a length of about 3 cm to about 8 cm.

4. Sanitary towel according to Claim 1, characterised in that the connection of the said lateral wings or flaps to the said top face of the said absorbent means is effected at one of the ends of the said absorbent means close to one of its transverse edges.

## Patentansprüche

1. Monatsbinde (1), die bestimmt ist, mit Unterwäsche zum Halten getragen zu werden, die ein Zwischenbeinteil aufweist, mit
a) einem absorbierenden Mittel (2) von länglicher Form, welches zwei gegenüberliegende längsgerichtete Ränder (21, 22), zwei gegenüberliegende quergerichtete Ränder (23, 24), eine für Körperflüssigkeiten durchlässige Oberseite für Berührung mit dem Körper und eine Unterseite zur Seite der Unterwäsche hin hat,
b) zwei seitlichen Flügeln (3) oder Seitenteilen (3), die sich entlang der längsgerichteten Ränder des absorbierenden Mittels erstrecken,
dadurch gekennzeichnet, daß die seitlichen Flügel oder Seitenteile eine Länge haben, die im wesentlichen gleich derjenigen des absorbierenden Mittels ist, das sie mit dem absorbierenden Mittel durch eine Verbindung an der Oberseite verbunden sind, und daß sie an jedem ihrer freien Enden mit einem Verbindungselement (4) versehen sind, welches ihre Befestigung an der Unterwäsche ermöglicht.

2. Monatsbinde gemäß Anspruch 1, gekennzeichnet durch die Tatsache, daß die Verbindung von jedem der seitlichen Flügel oder Seitenteile an der Oberseite des absorbierenden Mittels in dem zentralen Teil des absorbierenden Mittels in der Nähe der jeweiligen längsgerichteten Ränder des absorbierenden Mittels verwirklicht ist.

3. Monatsbinde gemäß Anspruch 2, gekennzeichnet durch die Tatsache, daß die Verbindung auf einer Länge von ungefähr 3 cm bis ungefähr 8 cm verwirklicht ist.

4. Monatsbinde gemäß Anspruch 1, gekennzeichnet durch die Tatsache, daß die Verbindung der seitlichen Flügel oder Seitenteile an der Oberseite des absorbierenden Mittels mit dem einen der Enden des absorbierenden Mittels in der Nähe des einen seiner quergerichteten Ränder verwirklicht ist.
